# EUROPEAN PATENT SPECIFICATION

(11) **EP 0 807 430 B1**
(45) Date of publication and mention of the grant of the patent: **03.04.2002**
(21) Application number: 97201426.0
(22) Date of filing: 12.05.1997
(51) Int. Cl.: A61K 9/00

(54) **Medicinal speciality for controlling the birth-rate of birds, and a method for its manufacture**
Medizinische Spezialität zur Kontrolle der Geburten-rate bei Vögeln und ein Verfahren zu deren Herstellung
Specialité médicale pour contrôler le taux de naissances des oiseaux et procédé de fabrication

(30) Priority: 16.05.1996 IT RE960035
(43) Date of publication of application: 19.11.1997
(73) Proprietor: Acme Drugs S.r.l., 42025 Cavriago (Emilia) (IT)
(72) Inventor: Predieri, Paolo, 42025 Cavriago (RE) (IT); Arduini, Lauro, 42025 Cavriago (RE) (IT)
(74) Representative: Corradini, Corrado

(56) References cited:
- DE-A- 4 118 550
- GB-A- 2 075 818
- US-A- 5 504 114
- MONTECUCCOLI G.: "The role of 25-hydroxycholecalciferol-1-hydroxylase in the responses of calcium absorption to the reproductive activity in birds" COMP. BIOCHEM. PHYSIOL., vol. 57, no. 3a, 1977, pages 335-339, XP002048983
- LEESON S: "The effect of graded levels of nicarbazin on reproductive performance of laying hens" CAN. JOURN. ANIM.SCI., vol. 69, no. 3, September 1989, pages 757-764, XP002048984
- JONES J E: "Reproduction responses of Broiler-Breeders to anticoccidial agents" POULT. SCI., vol. 69, no. 1, 1990, pages 27-36, XP002048985

## Description

This patent relates to a medicinal speciality for controlling the birth of birds in general, with particular reference to wild birds, such as pigeons, which nest in ever increasing number in inhabited centres.

The increase in the bird population in inhabited centres, especially of pigeons, creates both economical and hygiene problems.

Apart from the uncontrollable street soiling which considerably increases the expense of urban cleaning, of particular seriousness is the irreversible damage to monuments caused by their excrement, the components of which react with marble, stone and brick to cause microfissures, with consequent crumbling.

From the hygiene viewpoint the risks connected with fecal contamination of the environment are immediately apparent, with consequent wind dispersion of the dried and pulverized defecation, with its pathogenic germ load (especially salmonellosis).

It is therefore understandable that for some time, methods have been studied for controlling the wild bird population, especially pigeons, in inhabited centres, however these methods have so far not given satisfactory results, for the ensuing reasons.

The most ancient methods, such as periodical bird slaughtering, egg destruction close to brooding time, and the use of physical impediments to nest building, such as nets and repellent substances, have proved totally inadequate because of the vastness of the phenomenon.

Other known methods consist substantially of administering feeds containing hormone associations (estrogenic and/or progestational) or active principles of different origin which act via general toxicity mechanisms such as to hinder reproduction.

DE 41 18 550 A discloses a medicinal speciality of veterinary use for inhibiting the hatching of urban pigeons comprising a carrier and hormones.

US-A-5 504 114 discloses a method for controlling bird populations comprising administering to female birds conjugated linoleic acid.

The use of hormone products is increasingly less acceptable in that hormones (estrogenic, progestational) are substances which are very active also against man, and need to be used in quantities which constitute too high a risk for the health of the public.

For example, for controlling the wild pigeon population of a city such as Venice, 300 g per day of progesterone would have to be distributed over the territory during the entire pigeon copulation period.

Such a quantity would inevitably affect water sources or strata, with inevitable consequences for man.

The use of active principles the action mechanisms of which fall within the framework of general toxicity is always less acceptable, as this is now on account of animal well-being governed by civil law.

An object of the invention is firstly to provide a medicinal speciality which enables the birth-rate of birds which nest in urban centres to be controlled, or even nullified, without presenting problems for man, and with maximum respect for animal well-being.

A further object of the invention is to provide a medicinal speciality in a form which enables its easy dosing and administration.

A further object is to provide a medicinal speciality able to selectively and automatically exclude from its action those small birds, such as sparrows, starlings, blackbirds or the like, which may be protected by law.

Said objects are attained according to the invention by an active principle known as nicarbazin.

Up to the present time, this active principle has been known as a chicken feed additive for preventing a very common protozoan parasitotis, namely coccidiosis.

MONTECUCCOLI G.: 'The role of 25-hydroxycholecalciferol-1 -hydroxylase in the responses of calcium absorption to the reproductive activity in birds' COMP. BIOCHEM. PHYSIOL., vol. 57, no. 3a, 1977, pages 335-339, XP002048983; LEESON S: 'The effect of graded levels of nicarbazin on reproductive performance of laying hens' CAN. JOURN. ANIM.SCI., vol. 69, no. 3, September 1989, pages 757-764, XP002048984 or JONES J E: 'Reproduction responses of Broiler-Breeders to anticoccidial agents' POULT. SCI., vol. 69, no. 1, 1990, pages 27-36, XP002048985 disclose the effect of nicarbazin on the reduction of egg production in hens.

It has proved free of undesirable side-effects, it is non-toxic and neither does it produce acute or chronic organic lesions in pigeons.

Nicarbazin is insoluble in water, insoluble in fats and insoluble in common organic solvents, and is therefore devoid of any negative environmental impact, to the extent that the health authorities of the main countries allow its use as a chicken feed additive.

Nicarbazin has shown unexpected antifertility qualities, even in very small doses, and with perfectly controllable effects.

When added to the feed in amounts of between 200 ppm and 1000 ppm, its effects vary from a modest reduction in egg hatching to complete hatching inhibition.

According to the invention, this active principle is associated with a feed in the form of whole cereal grains, and by choosing a cereal with fairly large grains the active principle is prevented from being consumed by small birds.

In particular, the antifertility effect of nicarbazin on pigeons appears at very small doses, to the advantage of environmental protection.

Nicarbazin reduces bird fertility when added to the feed in an amount of 200 p.p.m. (parts per million).

An amount of 400 parts per million in the feed eliminates egg hatching.

Conveniently, the feed must contain about 800 p.p.m. of nicarbazin to ensure effective nicarbazin dosing even where the wild pigeons have partial alternative food sources available, and hence consume only a small quantity of feed.

Considering that an adult pigeon consumes about 30 g of food per day, the feed provides its antifertility effect even if consumed in a quantity of only 15 g per pigeon per day.

If the nicarbazin is associated with whole maize grains, it does not accidentally interfere with small bird species such as blackbirds, thrushes, starlings and sparrows, as it would if merely used in a simple pellet-type feed containing 800 p.p.m. of nicarbazin.

In this case a pellet obtained by compressing meal can easily break and be consumed by small birds. Moreover, when exposed to rain. a pellet may disintegrate, release the active principle and again be consumed by aviary birds, which are protected and of whom reproduction must not be impeded.

Small birds such as blackbirds, thrushes and sparrows are incapable of consuming whole maize grains.

According to the invention the medicinal speciality consists of maize seeds covered with nicarbazin in an amount of 800 p.p.m., all coated with a film which under normal atmospheric conditions is water-insoluble.

This ensures that the active principle cannot be washed out in the case of rain.

The film coating the maize seeds and containing the nicarbazin dissolves within the pigeon digestive system, so that the active principle becomes satisfactorily bioavailable.

The invention provides an extremely effective medicinal speciality of very small if not totally negligible environmental impact, so much so that, as stated, its active principle is normally used as an additive in chicken feed production.

It has a reversible effect, in that after 12-14 days from the last administration, laid eggs are normally fertile.

Because of its very low toxicity, it causes neither acute nor chronic lesions to birds.

The method of preparing a feed according to the invention is described hereinafter, it having the following composition referred to 100 grams of grains for oral use:

| COMPONENT NAME | QUANTITY (g) | FUNCTION | REFERENCE |
|---|---|---|---|
| Active component: | | | |
| NICARBAZIN | 0.080 | Inhibits pigeon egg fertility | |

| Other components: | | | |
|---|---|---|---|
| GELATIN TYPE B | 5.000 | Water-sol. coating | F.U.IX |
| WATER-REP. POLYMER | 0.500 | Water-repell. coating | |
| MAIZE GRAINS | 93.720 | Vehicle | |
| SODIUM CHLORIDE | 0.700 | Electrolyte | F.U.IX |

The medicinal speciality for veterinary use based on nicarbazin in microencapsulated form and carried by maize grains must be produced in a production department approved for the production of suspensions for oral use.

The temperature and humidity conditions of the department must be as follows:
- relative humidity: 55-65%
- ambient temperature: 20-25°C

It is preferable to produce standard batches weighing 1000 kg, for each of which the following starting substances are required:

| | |
|---|---|
| Nicarbazin | 0.800 kg |
| Gelatin type B (AL 280 Bloour) | 50.000 kg |
| Water-repellent polymer | 5.000 kg |
| Maize grains | 937.200 kg |
| Purified water | 100.000 kg |
| Electrolyte | 7.000 kg |
| Formaldehyde | |

The following equipment is required:
- Melter of 200 litre capacity (for example by DUMEK)
   - heating by resistance elements;
   - cooling by mains water;
   - circulation pump and thermostat;
   - impeller mixing;
- Biconical mixer of 4000 litre capacity
   - drying by resistance elements;
   - vacuum pump;
- Electronic weigher for weight control
- Electric welder for closing bags.

The steps in the manufacturing process are as follows:
- Component weighing
- Aqueous phase preparation
- Incorporating the micronized active principle
- pH variation by electrolytes
- Maize grain preparation in the biconical dryer
- Covering maize grains with the aqueous suspension
- Drying under vacuum and consolidating the microcapsules
- Covering with water-repellent polymer
- Drying
- Granule control.

Aqueous phase preparation. This is done in the following manner after the weighing operations:
100 kg of purified water are fed into the melter.
The melter is heated to a temperature of 35-37°C under agitation. 50 kg of gelatin of type B are added until completely dissolved.

Nicarbazin incorporation is then carried out as follows:
800 grams of micronized nicarbazin are added under agitation to the aqueous solution at a temperature not exceeding 40°C.
Under these conditions a suspension of active principle in a colloidal solution of water and gelatin is created.
Electrolytes in the form of sodium chloride and sodium hydroxide are added to the suspension until the solution reaches pH 8.5-9. The pH change causes coacervation (partial desolvation and concentration) of the colloid molecules, which are adsorbed about the particles of active principle to form a coating film.
The result is an aqueous suspension of granules of active principle microencapsulated in gelatin.

Maize spray-covering with microencapsulated nicarbazin is then carried out.
The maize in grain form contained in the biconical mixer is covered by spraying with the microencapsulated nicarbazin suspension.

Drying and consolidation are finally carried out during which the mixer is put under vacuum and the temperature raised to about 20-25°C to evaporate all the water of suspension.

To consolidate the microencapsulation of the active principle, the dried product is treated with formaldehyde by spraying it into the biconical mixer.

The maize grains treated in the aforegoing manner are then covered with water-repellent polymer following the aforedescribed procedures.

After complete drying under vacuum, the product can be packed.

The biconical mixer is opened and the granules are discharged into closable PVC containers of 200 kg capacity each. Sampling is then effected from these containers for quality control of the speciality in its final pharmaceutical form.

If the result is positive, the product is packed into aluminium bags.

The bags are sealed manually by a thermowelder of gripper type which welds the two bag edges together.

## Claims

1. A medicinal speciality of veterinary use for inhibiting the hatching of birds' eggs, **characterised by** comprising whole cereal grains coated with an insoluble film incorporating nicarbazin in amounts of between 200 p.p.m. and 1000 p.p.m.

2. A medicinal speciality as claimed in claim 1, **characterised in that** the insoluble film is composed of at least two layers, namely an inner water-soluble layer in contact with the grains and composed of a gelatin incorporating the nicarbazin, and an outer layer composed of a water-repellent polymer.

3. A medicinal speciality as claimed in claim 1, **characterised in that** the cereal is Indian corn or maize.

4. A medicinal speciality as claimed in claim 1, **characterised by** having the following weight composition per 1000 kg of product:
| | |
|---|---|
| Nicarbazin | 0.800 kg |
| Gelatin type B (Al 280 Bloour) | 50.000 kg |
| Water-repellent polymer | 5.000 kg |
| Maize grains | 937.200 kg |
| Purified water | 100.000 kg |
| Electrolyte (sodium chloride) | 7.000 kg |

5. A process for manufacturing the medicinal speciality claimed in claims 1 to 4, **characterised by** comprising the following steps:
- heating the purified water in a melter to a temperature of between 35 and 37°C, adding the gelatin and mixing until totally dissolved;
- adding micronized nicarbazin under continuous agitation, without exceeding 40°C;
- adding electrolytes to adjust the pH to between 8.5 and 9; to hence obtain active principle granules encapsulated in gelatin,. in aqueous suspension which is
- sprayed onto the maize in grain form contained in a biconical mixer; followed by
- drying, consisting of complete evaporation at a temperature of between 20 and 25°C;
- consolidating by spraying formaldehyde;
- coating with water-repellent polymer;
- final drying under vacuum.

## Patentansprüche

1. Medizinische Spezialität zum Gebrauch in der Veterinärmedizin zur Verhinderung des Legens von Vogeleiern,
**dadurch gekennzeichnet, daß**
sie ganze Getreidekörner umfaßt, die mit einem nicht löslichen Film mit Nicarbazin in einer Menge zwischen 200 ppm und 1000 ppm überzogen sind.

2. Medizinische Spezialität nach Anspruch 1,
**dadurch gekennzeichnet, daß**
der nicht lösliche Film aus mindestens zwei Schichten besteht, nämlich aus einer sich mit den Körnern in Kontakt befindlichen, inneren, wasserlöslichen Schicht, die aus das Nicarbazin enthaltender Gelatine besteht, und aus einer Außenschicht, die aus einem wasserabweisenden Polymer besteht.

3. Medizinische Spezialität nach Anspruch 1,
**dadurch gekennzeichnet, daß** das Getreide Mais ist.

4. Medizinische Spezialität nach Anspruch 1,
**dadurch gekennzeichnet, daß**
sie pro 1000 kg des Produktes die folgende Gewichtszusammensetzung aufweist:
| | |
|---|---|
| Nicarbazin | 0,800 kg |
| Gelatine Typ B (A1 280 Bloour) | 50,000 kg |
| Wasserweisendes Polymer | 5,000 kg |
| Maiskörner | 937,200 kg |
| Gereinigtes Wasser | 100,000 kg |
| Elektrolyt (Natriumchlorid) | 7,000 kg |

5. Verfahren zur Herstellung der medizinischen Spezialität nach Anspruch 1 bis 4,
**dadurch gekennzeichnet, daß**
es die folgenden Schritte umfaßt:
- Erhitzen des gereinigten Wassers in einem Schmelzer auf eine Temperatur zwischen 35 und 37°C, wobei die Gelatine hinzugegeben und solange gerührt wird, bis sie vollständig aufgelöst ist;
- Hinzugabe von micronisiertem Nicarbazin unter ständigem Rühren, ohne 40°C zu überschreiten;
- Hinzugabe von Elektrolyten zur Einstellung des pH-Wertes zwischen 8,5 und 9, um dadurch in Gelatine eingekapselte Wirkstoffkörnchen in einer wässrigen Lösung zu erhalten, die
- auf den in einem doppelkonischen Mixer in Form von Körnern enthaltenen Mais gesprüht wird, und nachfolgendes
- Trocknen, das in der vollständigen Verdunstung bei einer Temperatur zwischen 20 und 25°C besteht;
- Verfestigung durch Sprühen von Formaldehyd;
- Überziehen mit wasserabweisendem Polymer;
- Endgültiges Trocknen unter Vakuum.

## Revendications

1. Spécialité médicinale à usage vétérinaire pour empêcher l'éclosion d'oeufs d'oiseaux, **caractérisée en ce qu'**elle comprend des graines de céréale entières revêtues d'un film insoluble incorporant de la nicarbazine dans des quantités comprises entre 200 ppm et 1000 ppm.

2. Spécialité médicinale selon la revendication 1, **caractérisée en ce que** le film insoluble est constitué d'au moins deux couches, à savoir une couche intérieure soluble dans l'eau en contact avec les graines et qui est constituée d'une gélatine incorporant la nicarbazine, et une couche extérieure constituée d'un polymère imperméable à l'eau.

3. Spécialité médicinale selon la revendication 1, **caractérisée en ce que** la céréale est du blé d'Inde ou maïs.

4. Spécialité médicinale selon la revendication 1, **caractérisée en ce qu'**elle a la composition pondérale suivante pour 1000 kg de produit :
| | |
|---|---|
| nicarbazine | 0,800 kg |
| gélatine type B (Al 280 Bloour) | 50,000 kg |
| polymère imperméable à l'eau | 5,000 kg |
| maïs en grains | 937,200 kg |
| eau purifiée | 100,000 kg |
| électrolyte (chlorure de sodium) | 7,000 kg |

5. Procédé de fabrication d'une spécialité médicinale telle que revendiquée dans les revendications 1 à 4, **caractérisé en ce qu'**il comprend les étapes suivantes :
-- le chauffage de l'eau purifiée dans une cuve de fusion jusqu'à une température comprise entre 35 et 37°C, l'addition de la gélatine et le mélange jusqu'à dissolution totale ;
-- l'addition de nicarbazine micronisée sous agitation continue, sans dépasser la température de 40°C ;
-- l'addition d'électrolytes pour ajuster le pH à une valeur comprise entre 8,5 et 9 ;
-- de manière à obtenir ainsi des granules premiers actifs encapsulés dans la gélatine, dans une suspension aqueuse, laquelle est
-- projetée sur le maïs en grain contenu dans un appareil mélangeur à double conicité ; cette étape étant suivie par
-- un séchage, consistant à une évaporation complète à une température comprise entre 20 et 25°C ;
-- une consolidation par projection de formaldéhyde ;
-- un revêtement au moyen d'un polymère imperméable à l'eau ;
-- un séchage final sous vide.
